# EUROPEAN PATENT APPLICATION

(11) **EP 2 302 381 A1**
(43) Date of publication of application: **30.03.2011**
(21) Application number: 09164153.0
(22) Date of filing: 30.06.2009
(51) Int. Cl.: G01N 33/50

(54) **Gene signature for replicative senescence in cell cultures**

(71) Applicant: Ruprecht-Karls-Universität Heidelberg, 69117 Heidelberg (DE); Universität Graz, 8036 Graz (AT)
(72) Inventor: Bork, Simone, 40591 Düsseldorf (DE); Horn, Patrick, 69115 Heidelberg (DE); Ho, Anthony D., Prof.Dr., 69120 Heidelberg (DE); Schallmoser, Katharina, Dr., 8054 Graz (AT); Rohde, Eva, Dr., 8051 Graz (AT); Bartmann, Christina, Dr., 8054 Pirka (AT); Strunk, Dirk, Dr., 8062 Kumberg (AT); Wagner, Wolfgang, Dr., 52078 Aachen (DE)
(74) Representative: Herzog, Martin

(57) **Abstract**

The present invention relates to the field of diagnostics. Specifically, it relates to means and methods for determining the replicative senescence status of a cell culture and for assessing the suitability of a cell culture for therapeutic uses.

## Description

The present invention relates to the field of diagnostics. Specifically, it relates to means and methods for determining the replicative senescence status of a cell culture and for assessing the suitability of a cell culture for therapeutic uses.

Mesenchymal stem cells (MSC, also known as mesenchymal stromal cells) comprise a multipotent cell population that can differentiate into mesodermal cell lineages including osteocytes, chondrocytes and adipocytes. MSCs raise high expectations regarding cellular therapy and tissue engineering such as treatment of steroid refractory graft *versus* host disease (GVHD) or orthopaedic applications (e.g. bone defects). They can be isolated from a variety of tissues, by diverse separation protocols, in different culture media and under different growth conditions (Wagner and Ho, 2007, Mesenchymal stem cell preparations - comparing apples and oranges, Stem Cell Reviews and Reports, 3: 239-248). Usually, MSCs are separated by plastic adherence while non-adherent cells are removed by washing steps. All known isolation methods for MSCs result in heterogeneous cell populations. Reliable markers for the definition of multipotent subsets are still elusive (Ho et al., 2008, Heterogeneity of mesenchymal stromal cell preparations, Cytotherapy, 10: 320-330).

Molecular characterization of MSCs is further complicated by the fact that MSCs undergo changes in the course of in vitro culture expansion. All human somatic cells that can be propagated successfully in cell culture undergo replicative senescence (Smith and Pereira-Smith, 1996, Replicative senescence: Implications for in vivo aging and tumor suppression, Science, 273: 63-67). Within 20 to 50 population doublings (PDs), cells enlarge and become more granular. Cellular aging and replicative senescence of MSCs are influenced by their culture conditions. Colter and co-workers reported that single cellderived MSC clones can be expanded for up to 50 PDs if they are maintained at low density by repeated passaging, whereas cells stopped growing after 15 doublings if cultured at high cell density (Colter et al., 2001, Rapid expansion ofrecycling stem cells in cultures of plastic-adherent cells from human bone marrow, Proc. Natl. Acad. Sci. USA, 97: 3213-3218). Senescent cells remain metabolically active and can be maintained in this state for years. Replicative senescence does not only influence proliferation but also the differentiation potential of MSCs (Bonab et al., 2006, Aging of mesenchymal cells in vitro, BMC Cell Biology, 7: 14; Stenderup et al., 2003, Aging is associated with decreased maximal lifespan and accelerated senescence of bone marrow stromal cells, Bone, 33: 919-926; Fehrer and Lepperdinger, 2005, Mesenchymal stem cell aging, Exp. Gerontol., 40: 926-930; Baxter et al., 2004, Study of telomere length reveals rapid aging of human marrow stromal cells following invitro expansion, Stem Cells, 22: 675-682; Wagner et al., 2008, Replicative senescence of mesenchymal stem cells - a continuous and organized process, PLoS ONE, 5: e2213). Furthermore, replicative senescence affects the hematopoiesis supportive function of MSCs (Walenda et al., 2009, Co-culture with mesenchymal stromal cells increases proliferation and maintenance of hematopoietic progenitor cells, J Cell Mol Med, Epub ahead of print). MSCs cultured under the aegis of adult pHPL cease proliferation after less than 50 PDs as opposed to more than 60 PDs in FBS-driven cultures (Reinisch et al., 2007, Humanized system to propagate cord blood-derived multipotent mesnchymal stromal cells for clinical application, Regen. Med., 2: 371-382).

So far, there is no specific molecular marker for replicative senescence upon long-term culture. The enzyme lysosomal pH6 β-galactosidase (SA-β-gal) has been shown to be active in senescent human fibroblasts, but not in quiescent, pre-senescent or differentiated cells (Dimri GP et al.,, 1995, A biomarker that identifies senescent human cells in culture and in aging skin in vivo, Proc Natl Acad Sci U S A, 92: 9363-9367.) However, the staining procedure is not very standardized and can hardly be quantified to monitor the process of cellular aging.

The heterogeneity of isolated MSCs as well as the physiological changes induced by cell culture affects the explanatory power of experiments performed with these cells. Moreover, the suitability of MSCs for medical uses depends on the proliferation and differentiation potential of the cells. Thus, there is a need for means and methods which allow for a reliable characterization of MSCs with respect to their replicative senescence status.

Therefore, the present invention relates to a method for determining the replicative senescence status of a cell comprising the steps of
a) determining the amount of at least one of the gene products selected from the group consisting of PARG-1, CDKN2B, PTN and MCM3 in a sample comprising said cell; and
b) comparing each amount measured in step a) to a reference amount whereby the replicative senescence status is determined.

The term "replicative senescence status" refers to the potential of a cell to undergo further cell divisions and to differentiate into different cell types. The replicative senescence status of a cell depends on the duration of in vitro culture, on the culture conditions and on the age of the donor. A cell in a more advanced replicative senescence status has less differentiation potential compared to a cell in a less advanced replicative senescence status. Similarly, the cell in a more advanced replicative senescence status has a lower proliferation potential, i.e. it shows a decreased proliferation rate and the cell population derived from this cell will undergo less population doublings.

The term "gene products" refers to polynucleotides or polypeptides of the any one of the following genes PARG-1, CDKN2B, PTN or MCM3.

PARG1 (PTPL1-associated RhoGTPase activating protein 1; alternatively named ARHGAP29) mediates regulation of Rho-proteins. These proteins are involved in signal cascades from the cell membrane for control of cell adhesion, motility and cell morphology by modifications of the actin cytoskeleton.

CDKN2B (cycline dependent kinase inhibitor 2B) inhibits activation of cyclin dependent kinases and TGF-β induced G1-phase. This protein is an important tumour suppressor and associated with cell growth.

PTN (Pheiotrophin) plays an important role for survival and large scale expansion of human embryonic stem cells.

MCM3 (minichromosome maintenance complex 3) is an important factor for genomic replication and part of the pre-replication complex.

Polynucleotides representing the aforementioned genes comprise, preferably, nucleic acid sequences as defined by SEQ ID NOs: 1 (PARG-1), 2 (CDKN2B), 3 (PTN) or 4 (MCM3). Polypeptides encoded by the genes, preferably, have an amino acid sequences as defined by any one of SEQ ID NOs: 5 (PARG-1), 6 (CDKN2B), 7 (PTN) and 8 (MCM3).

Gene products as used herein also encompass variants of the aforementioned specific polynucleotides and polypeptides. Said variants may represent orthologs, paralogs or other homologs of the polynucleotide of as described above. The polynucleotide variants, preferably, comprise a nucleic acid sequence **characterized in that** the sequence can be derived from the aforementioned specific nucleic acid sequences shown in SEQ ID NO: 1 to SEQ ID NO: 4 by at least one nucleotide substitution, addition and/or deletion whereby the variant nucleic acid sequence shall still encode a polypeptide having the activity as specified above. Variants also encompass polynucleotides comprising a nucleic acid sequence which is capable of hybridizing to the aforementioned specific nucleic acid sequences, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6 × sodium chloride/sodium citrate (= SSC) at approximately 45°C, followed by one or more wash steps in 0.2 × SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1 to 5 × SSC (pH 7.2). If organic solvent is present in the abovementioned buffer, for example 50% formamide, the temperature under standard conditions is approximately 42°C. The hybridization conditions for DNA:DNA hybrids are preferably for example 0.1 × SSC and 20°C to 45°C, preferably between 30°C and 45°C. The hybridization conditions for DNA:RNA hybrids are preferably, for example, 0.1 × SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer-based amplification of DNA, i.e. using degenerated primers against conserved domains of the aforementioned polypeptides. Oligonucleotides suitable as PCR primers as well as suitable PCR conditions are described in the accompanying Examples. As a template, DNA or cDNA from bacteria, fungi, plants or animals may be used. Further, variants include polynucleotides comprising nucleic acid sequences which are at least 70%, at least 75%, at least 80%, at least 85%, at least 90%, at least 95%, at least 98% or at least 99% identical to the nucleic acid sequences shown in SEQ ID NO: 1 to SEQ ID NO: 4. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences shown in SEQ ID NO: 5 to SEQ ID NO: 8. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit [Needleman and Wunsch (J. Mol. Biol. 48; 443-453 (1970)) and Smith and Waterman (Adv. Appl. Math. 2; 482-489 (1981))], which are part of the GCG software packet [Genetics Computer Group, 575 Science Drive, Madison, Wisconsin, USA 53711 (1991)], are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

The polynucleotide is, preferably, DNA including cDNA or RNA. More preferably, it is cDNA or mRNA. The term encompasses single as well as double stranded polynucleotides. Moreover, comprised are also chemically modified polynucleotides including naturally occurring modified polynucleotides such as glycosylated or methylated polynucleotides.

Variant polypeptides have at least the same essential immunological properties as the specific polypeptides and offer the same diagnostic information. In particular, they share the same essential immunological properties, if they are detectable by the same specific assays referred to in this specification, e.g., by ELISA assays using polyclonal or monoclonal antibodies specifically recognizing the said polypeptides. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific polypeptides. The degree of identity between two amino acid sequences can be determined by algorithms as described above.

A gene product comprising a fragment of any of the aforementioned nucleic acid sequences or amino acid sequences is also encompassed as a gene product as described above. Accordingly, the gene product may comprise or consist of the domains of the aforementioned conferring the same diagnostic information or - in the case of a polypeptide - also the same immunological properties. A fragment as meant herein, preferably, comprises at least 50, at least 100, at least 250 or at least 500 consecutive nucleotides of any one of the aforementioned nucleic acid sequences or encodes an amino acid sequence comprising at least 20, at least 30, at least 50, at least 80, at least 100 or at least 150 consecutive amino acids of any one of the aforementioned amino acid sequences.

The term "amount" is, preferably the amount of a gene product. Preferably the amount is an absolute amount. More preferably, the amount is a relative amount. A relative amount of a gene product is, preferably, the amount of the gene product in proportion to a sample of a defined size. More preferably, a relative amount is the amount of one of the aforementioned gene products in proportion to the amount of the gene product of a house keeping gene. Preferred house keeping genes are the glyceraldehyde 3-phosphate dehydrogenase gene, 18s RNA and beta-actin.

A "reference amount" as referred to in the present application is the amount of the at least one of the aforementioned gene products in a cell or cell culture in a known replicative senescence status. Preferably, a known replicative senescence status is defined by the physiological properties of the cell which is used to determine the reference amount. Said physiological properties are, preferably, the differentiation and proliferation potential of the cell.

The replicative senescence status of a cell is, preferably, determined by comparing the amount of the at least one gene product in the sample with the amount of the same gene product(s) in a reference cell, i.e. a cell in a known replicative senescence status. An amount higher or lower than the reference amount is indicative of a more advanced or less advanced replicative senescence status while an essentially identical amount is indicative for a cell having the same replicative senescence status. Higher or lower amounts as used herein are, preferably, amounts which are statistically significant higher or lower. Whether this is the case can be determined by the skilled artisan without further ado by applying standard techniques of statistics such as Student's t- test or similar statistical tests.

More preferably, a range of reference amounts is derived from cells in different replicative senescence statuses, thus, enabling the determination of the replicative senescence status with a higher resolution. PARG1 and CDKN2B are up-regulated upon replicative senescence. PTN and MCM3 are down-regulated upon replicative senescence. Thus, a higher amount of the gene products of PARG-1 and CDNK2B is, preferably, indicative of a more advanced replicative senescence status. Conversely, a lower amount of the gene products of PTN and MCM3 is, preferably, indicative of a more advanced replicative senescence status. The differences of the amounts of the gene products of the amounts of the gene products are, preferably, statistically significant. Whether a difference is statistically significant can be determined by the statistical techniques described above.

The term "comparing" as used herein encompasses comparing the amount of the at least one gene product comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to determine the replicative senescence status of a cell. Therefore, the reference amount is to be chosen so that either a difference or a similarity in the compared amounts allows determining the replicative senescence status of a cell or a cell culture.

Determining the amount of any peptide or polypeptide referred to in this specification relates to measuring the amount or concentration, preferably semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal
- sometimes referred to herein as intensity signal - may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods which may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods.

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers.

First, binding of a ligand may be measured directly, e.g. by NMR or surface plasmon resonance.

Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured.

Third, the ligand may be coupled covalently or non-covalently to a label allowing detection and measurement of the ligand. Labelling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method.

Methods for determining the amount of a polynucleotide are well known to the person skilled in the art. Preferred methods are based on specific hybridization or specific amplification of the above described polynucleotides. Hybridization based methods are, preferably, northern blot, an RNAse protection assay, serial analysis of gene expression (SAGE), and microarrays. A preferred amplification based method is quantitative real time PCR (q-RT-PCR).

For northern blotting the mRNA of the cells is separated by electrophoresis. Then the separated nucleic acid sequences are transferred to a suitable membrane, typically nylon. An oligonucleotide complementary to a sequence comprised by one of the above described gene products (the probe) is then added to the membrane in a suitable buffer. The probe is labelled, preferably with radioactive isotopes or a chemiluminescent label. The intensity of the signal generated by the probe is a measure for the amount of the gene product. In an RNAse protection assay an oligonucleotide probe is hybridized to the mRNA of the cell. After that the single-stranded RNA sequences, i.e. those stretches of RNA which do not match the probe, are degraded enzymatically. SAGE is based on the fact that short stretches of a polynucleotide are sufficient for its unambiguous identification. From defined positions of the mRNA molecules oligonucleotides are cleaved. These oligonucleotides are then linked to form a concatamer, cloned into a vector and sequenced. The frequency of a specific tag indicates the strength of the expression of the respective gene. Microarrays comprise large numbers of oligonucleotides immobilized on a solid support, preferably a glass slide or a nylon membrane. These probes have, preferably, a length of 5 to 50, more preferably 20 to 30 nucleotides. Preferably, the probes correspond to genomic DNA or to cDNA. With the aid of a suitable analyzer, such as an automatic reader device, the amount of mRNA bound to the spots on the microarray is precisely measured, generating a profile of gene expression in the cell. Preferably, the mRNA is transcribed into cDNA before hybridization to the microarray. q-RT-PCR exploits the ability of suitable primers to specifically amplify a polynucleotide comprising binding sites for both primers. Preferably, the gene product is transcribed into cDNA in a first step. The cDNA is then used as template for the amplification with specific primers in q-RT-PCR. By determining the amount of PCR products after each cycle of replication the amount of template, and thus the amount of the mRNA of interest, in the sample can be determined.

Especially preferred is the use of cDNA microarrays as described in the Examples.

The term "cell" refers to an eukaryotic cell, more preferably to the cell of a mammal, even more preferably to the cell of a rat, cat, dog, monkey, pig, sheep, horse or human. Most preferably it relates to a human cell. It is to be understood that the term "cell" includes single cells as well as larger numbers of cells.

In a preferred embodiment of the present invention the cell is a mesenchymal stromal cell (in the art also known as mesenchymal stem cell). These cells are multipotent, i.e. they can differentiate into cells of diverse types, preferably chondrocytes, adipocytes and osteocytes. A mesenchymal stromal cell has, preferably, the immunophenotypic profile (HLA-AB⁺, CD13⁺, CD29⁺, CD73⁺, CD90⁺, CD105⁺, CD146⁺ and NLA-DR⁻, CD5⁻, CD10⁻, CD14⁻, CD31⁻, CD34⁻, CD45⁻, CD56⁻). Mesenchymal stromal cells are, preferably, isolated from bone marrow.

The term "sample" refers to a sample of separated cells or to a sample from a tissue or an organ. Tissue or organ samples may be obtained from any tissue or organ by, e.g., biopsy. Separated cells may be obtained from the body fluids or the tissues or organs by separating techniques such as centrifugation or cell sorting. It will e understood that the cell, tissue or organ derived sample comprises the cells which shall be subjected to the method of the present invention, i.e. the cells the replicative senescence status of which shall be determined. Preferably, cell-, tissue- or organ samples are obtained from those cells, tissues or organs which express or produce the peptides referred to herein. Also preferably, separated cells are obtained from in vitro cell cultures. Preferably, the sample comprises mesenchymal stromal cells as defined above.

Advantageously, the method of the present invention allows a more detailed characterization of a cell or cell culture regarding its potential for further proliferation and/or differentiation. As set forth above, it is very difficult to generate homogenous cultures of mesenchymal stromal cells. Furthermore, the properties of the cultures depend on the applied protocols for isolation and in vitro cell culture as well as on the duration of in vitro cultivation. Therefore, it is difficult to compare experimental results obtained with different cell cultures. The method of the present invention allows for the comparison of the replicative senescence status of different cells or cell cultures, thus enabling the standardization of experimental procedures. By comparing the amounts of at least one of the above mentioned gene products in at least two cells or cell cultures it is, furthermore, possible to identify to cell cultures in the same status of replicative senescence. Thus, cell cultures with comparable or even identical properties can be selected for the application in question. A standardization is especially desirable because mesenchymal stromal cells are not only used in laboratory experiments but also for medical applications. To ensure the safety and efficacy of treatment protocols the cells used in these protocols must have defined properties. The method of the present invention, thus, gives the scientist as well as the physician a measure for the quality of mesenchymal stromal cells used in research and therapy.

In a preferred embodiment of the present invention the cell has been freshly isolated from a donor. Preferably, a freshly isolated cell has been in vitro cultured for less than 5 population doublings. More preferably, a freshly isolated cell (i.e. a cell without intermittent cell culture) or a cell of a cell culture of about 5 PDs (population doublings) is analyzed.

In another preferred embodiment of the present invention the cell has been long term cultured. Preferably, a long term cultivated cell has been cultured in vitro for at least 13, 18 or 23 population doublings. There is evidence that under certain culture conditions (e.g. continuous growth in very low densities) retards the senescence process to a higher number of population doublings.

In yet another preferred embodiment of the present invention the amount of the gene products of at least 2, 3 or 4 genes is determined. In an especially preferred embodiment of the present invention the amount of the gene products of all 4 genes is determined.

It will be understood from the above that the present invention also provides for a method for determining whether cells in a first and a second sample have the same replicative senescence status comprising the steps of
a) determining the amount of at least one of the gene products selected from the group consisting of PARG-1, CDKN2B, PTN and MCM3 in the said first and second sample; and
b) comparing the amount measured in step a) in the first sample to the amount measured in step a) in the second sample whereby an essentially identical amount indicates that the cells comprised by the first and second sample have the same replicative senescence status.

Such a method can be used to compare the replicative senescence status of, e.g., cells of two or even more cell cultures prior to an experiment in order to avoid senescence caused influences on the experiment. It will be understood that the method referred to before can also be applied for comparing the replicative senescence status among more than two samples, i.e. a first, second, third, fourth, etc. sample.

Moreover, the present invention relates to a method for identifying a cell culture which is suitable for therapeutic uses comprising the steps of
a) determining the amount of at least one of the gene products selected from the group consisting of PARG-1, CDKN2B, PTN and MCM3 in a sample comprising said cell; and
b) comparing each amount measured in step a) to a reference amount whereby the replicative senescence status is determined.

A "cell culture suitable for therapeutic uses" is a cell culture comprising cells which can differentiate into the cell type that is required for the therapeutic use in question. Moreover, the proliferation potential of the cells must allow for the expansion of the cell line in vitro and for proliferation of the administered cells in vivo.

A reference amount for a cell culture suitable for therapeutic uses is, preferably, derived from a cell culture which has been applied successfully for the therapeutic use in question.

Moreover, the present invention relates to the use of at least one gene selected from the group consisting of PARG-1, CDKN2B, PTN and MCM3 for the determination of the replicative senescence status of a cell comprised by a sample.

Furthermore, the present invention relates to a device for determining the replicative senescence status of a cell comprising
a) an analysis unit for determining the amount of at least one of the gene products selected from the group consisting of PARG-1, CDKN2B, PTN and MCM3 in a sample comprising said cell; and
b) an evaluation unit for comparing each amount measured in step a) to a reference amount whereby the replicative senescence status is determined.

The term "device" as used herein relates to a system of means comprising at least the aforementioned means operatively linked to each other as to allow the determination of the replicative senescence status of a cell or cell culture. Preferred means for determining the amounts of the gene products of the present invention, and means for carrying out the comparison are disclosed above in connection with the method of the invention. How to link the means in an operating manner will depend on the type of means included into the device. For example, where an analysis unit for automatically determining the amount of the gene products of the present invention is applied, the data obtained by said automatically operating analysis unit can be processed by, e.g., a computer program in order to obtain the desired results. Preferably, the means are comprised by a single device in such a case.

Said device, preferably, includes an analyzing unit for the measurement of the amount of the gene products of the present invention in an applied sample and an evaluation unit for processing the resulting data. Preferably, the evaluation unit comprises a database with the stored reference amounts. Alternatively, where means such as test stripes are used for determining the amount of the gene products, the evaluation unit may comprise control stripes or tables allocating the determined amount to a reference amount. The test stripes are, preferably, coupled to a ligand which specifically binds to the gene products referred to herein. The strip or device, preferably, comprises means for detection of the binding of said gene products to the said ligand. Preferred means for detection are disclosed in connection with embodiments relating to the method of the invention above. In such a case, the analysis unit and the evaluation unit are operatively linked in that the user of the system brings together the result of the determination of the amount and the diagnostic or prognostic value thereof due to the instructions and interpretations given in a manual. The analysis unit and the evaluation unit may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of raw data which need interpretation by the clinician. Preferably, the output of the device is, however, processed, i.e. evaluated, raw data the interpretation of which does not require a clinician. Further preferred devices comprise the analyzing units/devices (e.g., biosensors, arrays, solid supports coupled to ligands specifically recognizing the gene product, Plasmon surface resonance devices, NMR spectrometers, mass-spectrometers etc.) or evaluation units/devices referred to above in accordance with the method of the invention.

Moreover, the present invention relates to a kit for determining the replicative senescence status of a cell comprising
a) an analysing unit for determining the amount of at least one of the gene products selected from the group consisting of PARG-1, CDKN2B, PTN and MCM3 in a sample comprising said cell; and
b) an evaluation unit for comparing each amount measured in step a) to a reference amount whereby the replicative senescence status is determined.

The term "kit" as used herein refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that the kit of the present invention is to be used for practising the methods referred to herein above. It is, preferably, envisaged that all components are provided in a ready-to-use manner for practising the methods referred to above. Further, the kit preferably contains instructions for carrying out the said methods. The instructions can be provided by a user's manual in paper- or electronic form. For example, the manual may comprise instructions for interpreting the results obtained when carrying out the aforementioned methods using the kit of the present invention.

Furthermore the present invention relates to a device for identifying a cell culture which is suitable for therapeutic uses comprising
a) an analysing unit for determining the amount of at least one of the gene products selected from the group consisting of PARG-1, CDKN2B, PTN and MCM3 in a sample comprising said cell; and
b) an evaluation unit for comparing each amount measured in step a) to a reference amount whereby the replicative senescence status is determined.

Finally, the present invention relates to a kit for identifying a cell culture which is suitable for therapeutic uses comprising
a) an analysing unit for determining the expression level of at least one of the genes selected from the group consisting of PARG-1, CDKN2B, PTN and MCM3 in a sample comprising said cell; and
b) an evaluation unit for comparing each amount measured in step a) to a reference amount whereby the replicative senescence status is determined.

The following examples are only intended to illustrate the present invention. They shall not limit the scope of the claims in any way.

### Example 1: Isolation of FBS-MSC and pHPL-MSC

Bone marrow aspirates (BM; maximum 13.5mL) were obtained in 5mL syringes preloaded with 500 IU of preservative-free Heparin (Biochrom AG, Berlin, Germany) in 2.5mL aliquots from healthy donors (n=3; age, 9, 27 and 36 years; female : male, 1:2) after written informed consent according to protocols approved by an institutional review board. After harvest, BM samples were diluted in medium without further manipulation. Cells were cultured in alpha-modified minimum essential medium (α-MEM, M4526; Sigma-Aldrich; St. Louis; MO) supplemented with 25mM HEPES (Sigma), 2mM L-Glutamine (Sigma), 100 U/mL Penicillin and 100µg/mL Streptomycin (Gibco Cell Culture, Invitrogen Corporation, Grand Island, NY), and either 10% FBS (Hyclone, Logan, UT) or 10% pHPL. pHPL was prepared from buffy coat-derived platelet rich plasma from at least 40 whole blood donations. Preservative-free Heparin (2 U/mL; Biochrom AG, Berlin, Germany) was added to the medium before pHPL supplementation to avoid coagulation. BM mononuclear cell (MNC) count was analyzed by automated blood count (Coulter Onyx; Beckman Coulter, Fullerton, CA) and cells were seeded at a density corresponding to 0.6 - 1.0 x 10⁴ MNC/cm² in 3 - 5 tissue culture flasks (Coming Inc., Acton, MA) and cultured at 5% CO₂, 37°C, 95% air humidity. Non-adherent cells were removed by a complete change of medium after 2-3 3 days. Twice weekly, 30% of the medium was replaced by fresh supplemented medium and cells were harvested before reaching confluence (between day 11 and 16) with 0.05% trypsin/0.7mM EDTA (1-5 min, 37°C; Sigma-Aldrich). Numbers of nucleated cells were determined using a hemocytometer as the mean of four measurements.

### Example 2: Large scale expansion of FBS-MSC and pHPL-MSC

MSCs derived from primary culture (passage zero; P0) were seeded in α-MEM/10% pHPL and α-MEM/10% FBS with a seeding density of 30/cm² on 1.0 to 2.5 m² culture area in four to ten 4-layered cell factories (CF-4; Nalge Nunc International, Naperville, IL) and cultured to reach confluence. Donor A MSCs were primarily cultured only in FBS-medium but were re-seeded in FBS and pHPL for P1. Partial (30%) medium change was performed twice weekly and MSCs were harvested at days 12 to 13 by trypsinization (passage 1; P1). MSCs derived from P1 were immediately re-seeded in α-MEM/10% pHPL and α-MEM/10% FBS in a density of 10-30 cells per cm² in 3 to 4 tissue culture flasks (Coming Inc., Acton, MA) corresponding to a culture area of 675 - 900 cm² and cultured at 5% CO₂, 37°C, 95% air humidity for 12 to 14 days until reaching confluence (passage 2; P2). MSCs were harvested with 0.05% trypsin/0.7mM EDTA (1 - 5 min, 37°C; Sigma-Aldrich) and counted. Cumulative PDs were calculated in relation to the initial CFU-F frequency.

### Example 3: Isolation and expansion of MSC-M1

For comparison we used MSCs that had been isolated in culture medium M1. Cells were isolated from bone marrow aspirates from healthy donors after written informed consent according to guidelines approved by the Ethic Committee on the Use of Human Subjects at the University of Heidelberg. The medium M1 consisted of 58% Dulbecco's Modified Eagles Medium-Low Glucose (DMEM-LG, Cambrex, Apen, Germany) and 40% MCDB201 (Sigma, Deisenhofen, Germany), 2% FBS (HyClone, Bonn, Germany), supplemented with 2mM L-Glutamine, 100 U/mL Pen/Strep (Cambrex), 1% insulin transferrin selenium, 1% linoleic acid bovine serum albumin, 10nM dexamethasone, 0.1mM L-ascorbic-acid-2-phosphate (Sigma, Hamburg, Germany), PDGF-BB and EGF (10ng/mL each, R&D Systems, Wiesbaden, Germany). Tissue culture flasks were coated with 10ng/mL fibronectin (Sigma) before use. MSC-M1 were harvested upon sub-confluent growth at a density of 70% and always re-plated at 10⁴ cells per cm². Thereby the cell density remained constant at a relatively high level throughout the culture.

### Example 4: Microarray analysis of FBS-MSC and pHPL-MSC

FBS-MSC and pHPL-MSC were harvested by trypsinization after each passage. RNA from at least 3 x 10⁶ MSCs was extracted using TRIZOL (Invitrogen, Carlsbad, CA, US) followed by RNeasy clean-up (QIAGEN). RNA quality was controlled using the RNA 6000 Pico LabChip kit (Agilent, Waldbronn, Germany) and quantified with a NanoDrop ND-1000 Spectrophotometer (Nanodrop Technologies, Wilmington, USA). Reverse transcription (oligo dT-T7 promoter primed) and labeling of cDNA was carried out using the RT labeling kit (Applied Biosystems, Foster City, CA, US) according to the manufacturer's instructions. Forty micrograms of total RNA per sample was used as a starting amount. For external control the Chemiluminescence RT Labeling kit (Applied Biosystems, Foster City, CA, US; Cat.Nr. 4340415) spike in controls of three bacterial genes (bioB, bioC, bioD) were used. Global gene expression profiles were analyzed using the ABI 1700 Genetic Analyzer Platform and the Human Genome Survey Microarray version 2.0 (Applied Biosystems, Foster City, CA, US). For intra-chip normalization the fully corrected signal associated with the probe was used. The chemiluminescence (CL) signal specific for the probe was normalized by the fluorescence (FL) signal divided by the feature integration aperture. Raw data and a combined transformed data are stored at the ArrayExpress database at the European Bioinformatics Institute; experiment name: Transcriptional profiling of mesenchymal stromal cells to reveal senescence-associated gene expression changes; ArrayExpress accession: E-MEXP-1997; user name: Reviewer_E-MEXP-1997; password: 1233614296357).

### Example 5: Analysis of senescence-associated gene expression in FBS-MSC and pHPL-MSC

Raw data of all independently processed samples were quantile normalized. Subsequently, ratios of differential gene expression were calculated in comparison to the corresponding sample in passage 0 (P1/P0 and P2/P0) and log₂ transformed. For subsequent analysis we only considered probe sets that were detected as a high quality signal (flag 0) in all 12 hybridizations. Lists of regulated genes were assembled using Significant Analysis of Microarrays (SAM) using the MultiExperiment Viewer (MeV, TM4) with a false discovery rate (FDR) < 5. Differentially expressed genes were further classified by GeneOnthology analysis using GoMiner software available at the National Center for Biotechnology Information. Representation in functional categories was analyzed by Fisher's Exact p-value test. Chromosomal distribution of differentially regulated genes was analyzed by Gene set enrichment analysis (GSEA).

### Example 6: Quantitative real-time PCR analysis

Quantification of mRNA expression for candidate genes was performed by quantitative real-time PCR (qRT-PCR) using the ABI PRISM® 7700HT Sequence Detection System Instrument (Applied Biosystems, Applera Deutschland GmbH, Darmstadt, Germany). Total RNA was reverse transcribed by using the high capacity cDNA reverse transcription kit (Applied Biosystems). Primers were obtained from Biospring (Frankfurt, Germany)

**Table 1: Primers used for q-RT-PCR**

| Gene | Forward primer | Reverse primer | Length of amplicon [nt] |
|---|---|---|---|
| GAPDH | SEQ ID NO: 9 | SEQ ID NO: 10 | 142 |
| PARG-1 | SEQ ID NO: 11 | SEQ ID NO: 12 | 309 |
| CDKN2B | SEQ ID NO: 13 | SEQ ID NO: 14 | 274 |
| PTN | SEQ ID NO: 15 | SEQ ID NO: 16 | 299 |
| MCM3 | SEQ ID NO: 17 | SEQ ID NO: 18 | 300 |

q-RT-PCR reactions were performed with the power SYBR® green PCR master mix in a MicroAmp optical 96-well reaction plate with a ABI PRISM® 7700HT sequence detector (Applied Biosystems) according to the manufacturer's instructions. Gene expression levels were normalized to GAPDH expression, which was used as a housekeeping gene.

The following mean gene expression changes were observed between the 2^{nd} (7 to 12 cumulative population doubling) and senescent passages (20 to 30 cumulative population doublings:

| | |
|---|---|
| PARG-1 : | about 7-fold up-regulated |
| CDKN2B : | about 6-fold up-regulated |
| PTN : | about 13-fold down-regulated |
| MCM3: | about 4-fold down-regulated |

## Claims

1. A method for determining the replicative senescence status of a cell comprising the steps of
a) determining the amount of at least one of the gene products selected from the group consisting of PARG-1, CDKN2B, PTN and MCM3 in a sample comprising said cell; and
b) comparing each amount measured in step a) to a reference amount whereby the replicative senescence status is determined.

2. The method of claim 1, wherein the cell has been freshly isolated from a donor.

3. The method of claim 1, wherein the cell has been long term cultured.

4. The method of any of claims 1 to 3, wherein the cell is a mesenchymal stromal cell.

5. The method of any one of claims 1 to 4, wherein the amount of all 4 gene products is determined.

6. A method for identifying a cell culture which is suitable for therapeutic uses comprising the steps of
a) determining the amount of at least one of the gene products selected from the group consisting of PARG-1, CDKN2B, PTN and MCM3 in a sample comprising said cell; and
b) comparing each amount measured in step a) to a reference amount whereby the replicative senescence status is determined.

7. The method of claim 6, wherein the cell culture comprises mesenchymal stromal cells.

8. The method of claim 6 or 7, wherein the amounts of all 4 gene products are determined.

9. Use of at least one gene selected from the group consisting of PARG-1, CDKN2B, PTN and MCM3 for the determination of the replicative senescence status of a cell comprised by a sample.

10. A device for determining the replicative senescence status of a cell comprising
a) an analysis unit for determining the amount of at least one of the gene products selected from the group consisting of PARG-1, CDKN2B, PTN and MCM3 in a sample comprising said cell; and
b) an evaluation unit for comparing each amount measured in step a) to a reference amount whereby the replicative senescence status is determined.

11. A kit for determining the replicative senescence status of a cell comprising
a) an analysing unit for determining the amount of at least one of the gene products selected from the group consisting of PARG-1, CDKN2B, PTN and MCM3 in a sample comprising said cell; and
b) an evaluation unit for comparing each amount measured in step a) to a reference amount whereby the replicative senescence status is determined.

12. A device for identifying a cell culture which is suitable for therapeutic uses comprising
a) an analysing unit for determining the amount of at least one of the gene products selected from the group consisting of PARG-1, CDKN2B, PTN and MCM3 in a sample comprising said cell; and
b) an evaluation unit for comparing each amount measured in step a) to a reference amount whereby the replicative senescence status is determined.

13. A kit for identifying a cell culture which is suitable for therapeutic uses comprising
a) an analysing unit for determining the expression level of at least one of the genes selected from the group consisting of PARG-1, CDKN2B, PTN and MCM3 in a sample comprising said cell; and
b) an evaluation unit for comparing each amount measured in step a) to a reference amount whereby the replicative senescence status is determined.


SEQUENCE LISTING

<110>  Universitätsklinikum Heidelberg

<120>  Gene signature for replicative senescence in cell cultures

<130>  UH66509EP

<160>  18

<170>  PatentIn version 3.5

<210>  1
<211>  3786
<212>  DNA
<213>  Homo sapiens

<400>  1
atgattgctc acaaacagaa aaagacaaag aaaaaacgtg cttgggcatc aggtcaactc      60

tctactgata ttacaacttc tgaaatgggg ctcaagtcct taagttccaa ctctattttt     120

gatccggatt acatcaagga gttggtgaat gatatcagga agttctccca catgttacta     180

tatttgaaag aagccatatt ttcagactgt tttaaagaag ttattcatat acgtctagag     240

gaactgctcc gtgttttaaa gtctataatg aataaacatc agaacctcaa ttctgttgat     300

cttcaaaatg ctgcagaaat gctcactgca aaagtgaaag ctgtgaactt cacagaagtt     360

aatgaagaaa acaaaaacga tctcttccag gaagtgtttt cttctattga aactttggca     420

tttacctttg gaaatatcct tacaaacttc cttatgggag atgtaggcaa tgattcatta     480

ttgcgactgc ctgtttctcg agaaactaag tcgtttgaaa atgtttctgt ggaatcagtg     540

gactcatcca gtgaaaaagg aaatttttcc cctttagaac tagacaacgt gctgttaaag     600

aacactgact ctatcgagct ggctttgtca tatgctaaaa cttggtcaaa atatactaag     660

aacatagttt catgggttga aaaaaagctt aacttggaat tggagtccac tagaaatatg     720

gtcaagttgg cagaggcaac tagaactaac attggaattc aggagttcat gccactgcag     780

tctctgttta ctaatgctct tcttaatgat atagaaagca gtcacctttt acaacaaaca     840

attgcagctc tccaggctaa caaatttgtg cagcctctac ttggaaggaa aaatgaaatg     900

gaaaaacaaa ggaaagaaat aaaagagctt tggaaacagg agcaaaataa aatgcttgaa     960

gcagagaatg ctctcaaaaa ggcaaaatta ttatgcatgc aacgtcaaga tgaatatgag    1020

aaagcaaagt cttccatgtt tcgtgcagaa gaggagcatc tgtcttcaag tggcggatta    1080

gcaaaaaatc tcaacaagca actagaaaaa aagcgaaggt tggaagagga ggctctccaa    1140

aaagtagaag aagcaaatga actttacaaa gtttgtgtga caaatgttga agaaagaaga    1200

aatgatctag aaaataccaa aagagaaatt ttagcacaac tccggacact tgttttccag    1260

tgtgatctta cccttaaagc tgtaacagtt aacctcttcc acatgcagca tctgcaggct    1320

gcttcccttg cagacagttt acagtctctc tgtgatagtg ccaaactcta tgacccaggc    1380

caagagtaca gtgaatttgt caaggccaca aattcaactg aagaagaaaa agttgatgga    1440

aatgtaaata aacatttaaa tagttcccaa ccttcaggat ttggacctgc caactcttta    1500

gaggatgttg tacgccttcc tgacagttct aataaaattg aagaggacag atgctctaac    1560

agtgcagata taacaggtcc ttcctttata agatcatgga catttgggat gtttagtgat 1620
tctgagagca ctggagggag cagcgaatct agatctctgg attcagaatc tataagtcca 1680
ggagactttc atcgaaaact tccacgaaca ccatccagtg gaactatgtc ctctgcagat 1740
gatctagatg aaagagagcc accttcccct tcagaaactg gacccaattc ccttggaaca 1800
tttaagaaaa cattgatgtc aaaggcagct ctcacacaca agtttcgcaa attgagatcc 1860
cccacgaaat gtagggattg tgaaggcatt gtagtgttcc aaggtgttga atgtgaagag 1920
tgtctccttg tttgtcatcg aaagtgtttg gaaaatttag tcattatttg tggtcatcag 1980
aaacttccag gaaaaataca cttatttgga gcagaattca cacaagttgc aaaaaaggaa 2040
ccagatggta tcccttttat actcaaaata tgtgcctcag agattgaaaa tagagctttg 2100
tgtctacagg gaatttatcg tgtgtgtgga aacaaaataa aaactgaaaa attgtgtcaa 2160
gctttggaaa atggaatgca cttggtagat atttcagaat ttagttcaca tgatatctgt 2220
gacgtcttga aattatacct tcggcagctc ccagaaccat ttattttatt tcgattgtac 2280
aaggaattta tagaccttgc aaaagagatc caacatgtaa atgaagaaca agagacaaaa 2340
aagaatagtc ttgaagacaa aaaatggcca aatatgtgta tagaaataaa ccgaattctt 2400
ctaaaaagca aagaccttct aagacaattg ccagcatcaa attttaacag tcttcatttc 2460
cttatagtac atctaaagcg ggtagtagat catgcagaag aaaacaagat gaactccaaa 2520
aacttggggg tgatatttgg accaagtctc attaggccaa ggcccacaac tgctcctatc 2580
accatctcct cccttgcaga gtattcaaat caagcacgct tggtagagtt tctcattact 2640
tactcacaga agatcttcga tgggtcccta caaccacaag atgttatgtg tagcataggt 2700
gttgttgatc aaggctgttt tccaaagcct ctgttatcac cagaagaaag agacattgaa 2760
cgttccatga agtcactatt tttttcttca aaggaagata tccatacttc agagagtgaa 2820
agcaaaattt ttgaacgagc tacatcattt gaggaatcag aacgcaagca aaatgcgtta 2880
ggaaaatgtg atgcatgtct cagtgacaaa gcacagttgc ttctagacca agaggctgaa 2940
tcagcatccc aaaagataga agatggtaaa acccctaagc cactttctct gaaatctgat 3000
aggtcaacaa acaatgtgga gaggcatact ccaaggacca agattagacc tgtaagtttg 3060
cctgtagata gactacttct tgcaagtcct cctaatgaga gaaatggcag aaatatggga 3120
aatgtaaatt tagacaagtt ttgcaagaat cctgcctttg aaggagttaa tagaaaagac 3180
gctgctacta ctgtttgttc caaatttaat ggctttgacc agcaaactct acagaaaatt 3240
caggacaaac agtatgaaca aaacagccta actgccaaga ctacaatgat catgcccagt 3300
gcactccagg aaaaaggagt gacaacaagc ctccagatta gtggggacca ttctatcaat 3360
gccactcaac ccagtaagcc atatgcagag ccagtcaggt cagtgagaga ggcatctgag 3420
agacggtctt cagattccta ccctctcgct cctgtcagag cacccagaac actgcagcct 3480
caacattgga caacatttta taaaccacat gctcccatca tcagtatcag ggggaatgag 3540
gagaagccag cttcaccctc agcagcagtg cctcctggca cagatcacga tccccacggt 3600

ctcgtggtga agtcaatgcc agacccagac aaagcatcag cttgtcctgg gcaagcaact 3660

ggtcaaccta aagaagactc tgaggagctt ggcttgcctg atgtgaatcc aatgtgtcag 3720

agaccaaggc taaaacgaat gcaacagttt gaagacctcg aaggtgaaat tccacaattt 3780

gtgtag 3786

<210> 2
<211> 417
<212> DNA
<213> Homo sapiens

<400> 2
atgcgcgagg agaacaaggg catgcccagt gggggcggca gcgatgaggg tctggccagc 60

gccgcggcgc ggggactagt ggagaaggtg cgacagctcc tggaagccgg cgcggatccc 120

aacggagtca accgtttcgg gaggcgcgcg atccaggtca tgatgatggg cagcgcccgc 180

gtggcggagc tgctgctgct ccacggcgcg gagcccaact gcgcagaccc tgccactctc 240

acccgaccgg tgcatgatgc tgcccgggag ggcttcctgg acacgctggt ggtgctgcac 300

cgggccgggg cgcggctgga cgtgcgcgat gcctggggtc gtctgcccgt ggacttggcc 360

gaggagcggg gccaccgcga cgttgcaggg tacctgcgca cagccacggg ggactga 417

<210> 3
<211> 507
<212> DNA
<213> Homo sapiens

<400> 3
atgcaggctc aacagtacca gcagcagcgt cgaaaatttg cagctgcctt cttggcattc 60

attttcatac tggcagctgt ggatactgct gaagcaggga agaaagagaa accagaaaaa 120

aaagtgaaga agtctgactg tggagaatgg cagtggagtg tgtgtgtgcc caccagtgga 180

gactgtgggc tgggcacacg ggagggcact cggactggag ctgagtgcaa gcaaaccatg 240

aagacccaga gatgtaagat cccctgcaac tggaagaagc aatttggcgc ggagtgcaaa 300

taccagttcc aggcctgggg agaatgtgac ctgaacacag ccctgaagac cagaactgga 360

agtctgaagc gagccctgca caatgccgaa tgccagaaga ctgtcaccat ctccaagccc 420

tgtggcaaac tgaccaagcc caaacctcaa gcagaatcta agaagaagaa aaaggaaggc 480

aagaaacagg agaagatgct ggattaa 507

<210> 4
<211> 2427
<212> DNA
<213> Homo sapiens

<400> 4
atggcgggta ccgtggtgct ggacgatgtg gagctgcggg aggctcagag agattacctg 60

gacttcctgg acgacgagga agaccaggga atttatcaga gcaaagttcg ggagctgatc 120

agtgacaacc aataccggct gattgtcaat gtgaatgacc tgcgcaggaa aaacgagaag 180

agggctaacc ggcttctgaa caatgccttt gaggagctgg ttgccttcca gcgggcctta 240

aaggattttg tggcctccat tgatgctacc tatgccaagc agtatgagga gttctacgta 300
ggactggaag gcagctttgg ctccaagcac gtctccccgc ggactcttac ctcctgcttc 360
ctcagctgtg tggtctgtgt ggagggcatt gtcactaaat gttctctagt tcgtcccaaa 420
gtcgtccgca gtgtccacta ctgtcctgct actaagaaga ccatagagcg acgttattct 480
gatctcacca ccctggtggc ctttccctcc agctctgtct atcctaccaa ggatgaggag 540
aacaatcccc ttgagacaga atatggcctt tctgtctaca aggatcacca gaccatcacc 600
atccaggaga tgccggagaa ggccccagcc ggccagctcc cccgctctgt ggacgtcatt 660
ctggatgatg acttggtgga taaagcgaag cctggtgacc gggttcaggt ggtgggaacc 720
taccgttgcc ttcctggaaa gaagggaggc tacacctctg ggaccttcag gactgtcctg 780
attgcctgta atgttaagca gatgagcaag gatgctcagc cctctttctc tgctgaggat 840
atagccaaga tcaagaagtt cagtaaaacc cgatccaagg atatctttga ccagctggcc 900
aagtcattgg ccccaagtat ccatgggcat gactatgtca agaaagcaat cctctgcttg 960
ctcttgggag gggtggaacg agacctagaa aatggcagcc acatccgtgg ggacatcaat 1020
attcttctaa taggagaccc atccgttgcc aagtctcagc ttctgcggta tgtgctttgc 1080
actgcacccc gagctatccc caccactggc cggggctcct ctggagtggg tctgacggct 1140
gctgtcacca cagaccagga aacaggagag cgccgtctgg aagcaggggc catggtcctg 1200
gctgaccgag gcgtggtttg cattgatgaa tttgacaaaa tgtctgacat ggatcgcaca 1260
gccatccatg aagtgatgga gcagggtcga gtgaccattg ccaaggctgg catccatgct 1320
cggctgaatg cccgctgcag tgttttggca gctgccaacc ctgtctacgg caggtatgac 1380
cagtataaga ctccaatgga gaacattggg ctacaggact cactgctgtc acgatttgac 1440
ttgctcttca tcatgctgga tcagatggat cctgagcagg atcgggagat ctcagaccat 1500
gtccttcgga tgcaccgtta cagagcacct ggggagcagg atggcgatgc tatgcccttg 1560
ggtagtgctg tggatatcct ggccacagat gatcccaact ttagccagga agatcagcag 1620
gacacccaga tttatgagaa gcatgacaac cttctacatg ggaccaagaa gaaaaaggag 1680
aagatggtga gtgcagcatt catgaagaag tacatccatg tggccaaaat catcaagcct 1740
gtcctgacac aggagtcggc cacctacatt gcagaagagt attcacgcct gcgcagccag 1800
gatagcatga gctcagacac cgccaggaca tctccagtta cagcccgaac actggaaact 1860
ctgattcgac tggccacagc ccatgcgaag gcccgcatga gcaagactgt ggacctgcag 1920
gatgcagagg aagctgtgga gttggtccag tatgcttact ttaagaaggt tctggagaag 1980
gagaagaaac gtaagaagcg aagtgaggat gaatcagaga cagaagatga agaggagaaa 2040
agccaagagg accaggagca gaagaggaag agaaggaaga ctcgccagcc agatgccaaa 2100
gatggggatt catacgaccc ctatgacttc agtgacacag aggaggaaat gcctcaagta 2160
cacactccaa agacggcaga ctcacaggag accaaggaat cccagaaagt ggagttgagt 2220
gaatccaggt tgaaggcatt caaggtggcc ctcttggatg tgttccggga agctcatgcg 2280
cagtcaatcg gcatgaatcg cctcacagaa tccatcaacc gggacagcga agagcccttc 2340

tcttcagttg agatccaggc tgctctgagc aagatgcagg atgacaatca ggtcatggtg 2400

tctgagggca tcatcttcct catctga 2427

<210> 5
<211> 1261
<212> PRT
<213> Homo sapiens

<400> 5

Met Ile Ala His Lys Gln Lys Lys Thr Lys Lys Lys Arg Ala Trp Ala
1               5                   10                  15

Ser Gly Gln Leu Ser Thr Asp Ile Thr Thr Ser Glu Met Gly Leu Lys
            20                  25                  30

Ser Leu Ser Ser Asn Ser Ile Phe Asp Pro Asp Tyr Ile Lys Glu Leu
        35                  40                  45

Val Asn Asp Ile Arg Lys Phe Ser His Met Leu Leu Tyr Leu Lys Glu
    50                  55                  60

Ala Ile Phe Ser Asp Cys Phe Lys Glu Val Ile His Ile Arg Leu Glu
65                  70                  75                  80

Glu Leu Leu Arg Val Leu Lys Ser Ile Met Asn Lys His Gln Asn Leu
                85                  90                  95

Asn Ser Val Asp Leu Gln Asn Ala Ala Glu Met Leu Thr Ala Lys Val
            100                 105                 110

Lys Ala Val Asn Phe Thr Glu Val Asn Glu Glu Asn Lys Asn Asp Leu
        115                 120                 125

Phe Gln Glu Val Phe Ser Ser Ile Glu Thr Leu Ala Phe Thr Phe Gly
    130                 135                 140

Asn Ile Leu Thr Asn Phe Leu Met Gly Asp Val Gly Asn Asp Ser Leu
145                 150                 155                 160

Leu Arg Leu Pro Val Ser Arg Glu Thr Lys Ser Phe Glu Asn Val Ser
                165                 170                 175

Val Glu Ser Val Asp Ser Ser Ser Glu Lys Gly Asn Phe Ser Pro Leu
            180                 185                 190

Glu Leu Asp Asn Val Leu Leu Lys Asn Thr Asp Ser Ile Glu Leu Ala
        195                 200                 205

Leu Ser Tyr Ala Lys Thr Trp Ser Lys Tyr Thr Lys Asn Ile Val Ser
    210                 215                 220

Trp Val Glu Lys Lys Leu Asn Leu Glu Leu Glu Ser Thr Arg Asn Met
225                 230                 235                 240

Val Lys Leu Ala Glu Ala Thr Arg Thr Asn Ile Gly Ile Gln Glu Phe
                245                 250                 255

Met Pro Leu Gln Ser Leu Phe Thr Asn Ala Leu Leu Asn Asp Ile Glu
            260                 265                 270

Ser Ser His Leu Leu Gln Gln Thr Ile Ala Ala Leu Gln Ala Asn Lys
        275                 280                 285

Phe Val Gln Pro Leu Leu Gly Arg Lys Asn Glu Met Glu Lys Gln Arg
    290                 295                 300

Lys Glu Ile Lys Glu Leu Trp Lys Gln Glu Gln Asn Lys Met Leu Glu
305                 310                 315                 320

Ala Glu Asn Ala Leu Lys Lys Ala Lys Leu Leu Cys Met Gln Arg Gln
                325                 330                 335

Asp Glu Tyr Glu Lys Ala Lys Ser Ser Met Phe Arg Ala Glu Glu Glu
            340                 345                 350

His Leu Ser Ser Ser Gly Gly Leu Ala Lys Asn Leu Asn Lys Gln Leu
        355                 360                 365

Glu Lys Lys Arg Arg Leu Glu Glu Glu Ala Leu Gln Lys Val Glu Glu
    370                 375                 380

Ala Asn Glu Leu Tyr Lys Val Cys Val Thr Asn Val Glu Glu Arg Arg
385                 390                 395                 400

Asn Asp Leu Glu Asn Thr Lys Arg Glu Ile Leu Ala Gln Leu Arg Thr
                405                 410                 415

Leu Val Phe Gln Cys Asp Leu Thr Leu Lys Ala Val Thr Val Asn Leu
            420                 425                 430

Phe His Met Gln His Leu Gln Ala Ala Ser Leu Ala Asp Ser Leu Gln
        435                 440                 445

Ser Leu Cys Asp Ser Ala Lys Leu Tyr Asp Pro Gly Gln Glu Tyr Ser
    450                 455                 460

Glu Phe Val Lys Ala Thr Asn Ser Thr Glu Glu Glu Lys Val Asp Gly
465                 470                 475                 480

Asn Val Asn Lys His Leu Asn Ser Ser Gln Pro Ser Gly Phe Gly Pro
                485                 490                 495

Ala Asn Ser Leu Glu Asp Val Val Arg Leu Pro Asp Ser Ser Asn Lys

            500                 505                 510

Ile Glu Glu Asp Arg Cys Ser Asn Ser Ala Asp Ile Thr Gly Pro Ser
        515                 520                 525

Phe Ile Arg Ser Trp Thr Phe Gly Met Phe Ser Asp Ser Glu Ser Thr
    530                 535                 540

Gly Gly Ser Ser Glu Ser Arg Ser Leu Asp Ser Glu Ser Ile Ser Pro
545                 550                 555                 560

Gly Asp Phe His Arg Lys Leu Pro Arg Thr Pro Ser Ser Gly Thr Met
                565                 570                 575

Ser Ser Ala Asp Asp Leu Asp Glu Arg Glu Pro Pro Ser Pro Ser Glu
            580                 585                 590

Thr Gly Pro Asn Ser Leu Gly Thr Phe Lys Lys Thr Leu Met Ser Lys
        595                 600                 605

Ala Ala Leu Thr His Lys Phe Arg Lys Leu Arg Ser Pro Thr Lys Cys
    610                 615                 620

Arg Asp Cys Glu Gly Ile Val Val Phe Gln Gly Val Glu Cys Glu Glu
625                 630                 635                 640

Cys Leu Leu Val Cys His Arg Lys Cys Leu Glu Asn Leu Val Ile Ile
                645                 650                 655

Cys Gly His Gln Lys Leu Pro Gly Lys Ile His Leu Phe Gly Ala Glu
            660                 665                 670

Phe Thr Gln Val Ala Lys Lys Glu Pro Asp Gly Ile Pro Phe Ile Leu
        675                 680                 685

Lys Ile Cys Ala Ser Glu Ile Glu Asn Arg Ala Leu Cys Leu Gln Gly
    690                 695                 700

Ile Tyr Arg Val Cys Gly Asn Lys Ile Lys Thr Glu Lys Leu Cys Gln
705                 710                 715                 720

Ala Leu Glu Asn Gly Met His Leu Val Asp Ile Ser Glu Phe Ser Ser
                725                 730                 735

His Asp Ile Cys Asp Val Leu Lys Leu Tyr Leu Arg Gln Leu Pro Glu
            740                 745                 750

Pro Phe Ile Leu Phe Arg Leu Tyr Lys Glu Phe Ile Asp Leu Ala Lys
        755                 760                 765

Glu Ile Gln His Val Asn Glu Glu Gln Glu Thr Lys Lys Asn Ser Leu
    770                 775                 780

Glu Asp Lys Lys Trp Pro Asn Met Cys Ile Glu Ile Asn Arg Ile Leu
785                 790                 795                 800

Leu Lys Ser Lys Asp Leu Leu Arg Gln Leu Pro Ala Ser Asn Phe Asn
                805                 810                 815

Ser Leu His Phe Leu Ile Val His Leu Lys Arg Val Val Asp His Ala
            820                 825                 830

Glu Glu Asn Lys Met Asn Ser Lys Asn Leu Gly Val Ile Phe Gly Pro
        835                 840                 845

Ser Leu Ile Arg Pro Arg Pro Thr Thr Ala Pro Ile Thr Ile Ser Ser
    850                 855                 860

Leu Ala Glu Tyr Ser Asn Gln Ala Arg Leu Val Glu Phe Leu Ile Thr
865                 870                 875                 880

Tyr Ser Gln Lys Ile Phe Asp Gly Ser Leu Gln Pro Gln Asp Val Met
                885                 890                 895

Cys Ser Ile Gly Val Val Asp Gln Gly Cys Phe Pro Lys Pro Leu Leu
            900                 905                 910

Ser Pro Glu Glu Arg Asp Ile Glu Arg Ser Met Lys Ser Leu Phe Phe
        915                 920                 925

Ser Ser Lys Glu Asp Ile His Thr Ser Glu Ser Glu Ser Lys Ile Phe
    930                 935                 940

Glu Arg Ala Thr Ser Phe Glu Glu Ser Glu Arg Lys Gln Asn Ala Leu
945                 950                 955                 960

Gly Lys Cys Asp Ala Cys Leu Ser Asp Lys Ala Gln Leu Leu Leu Asp
                965                 970                 975

Gln Glu Ala Glu Ser Ala Ser Gln Lys Ile Glu Asp Gly Lys Thr Pro
            980                 985                 990

Lys Pro Leu Ser Leu Lys Ser Asp  Arg Ser Thr Asn Asn  Val Glu Arg
        995                 1000                 1005

His Thr  Pro Arg Thr Lys Ile  Arg Pro Val Ser Leu  Pro Val Asp
    1010                 1015                 1020

Arg Leu  Leu Leu Ala Ser Pro  Pro Asn Glu Arg Asn  Gly Arg Asn
    1025                 1030                 1035

Met Gly  Asn Val Asn Leu Asp  Lys Phe Cys Lys Asn  Pro Ala Phe
    1040                 1045                 1050

Glu Gly Val Asn Arg Lys Asp Ala Ala Thr Thr Val Cys Ser Lys
    1055                    1060                    1065

Phe Asn Gly Phe Asp Gln Gln Thr Leu Gln Lys Ile Gln Asp Lys
    1070                    1075                    1080

Gln Tyr Glu Gln Asn Ser Leu Thr Ala Lys Thr Thr Met Ile Met
    1085                    1090                    1095

Pro Ser Ala Leu Gln Glu Lys Gly Val Thr Thr Ser Leu Gln Ile
    1100                    1105                    1110

Ser Gly Asp His Ser Ile Asn Ala Thr Gln Pro Ser Lys Pro Tyr
    1115                    1120                    1125

Ala Glu Pro Val Arg Ser Val Arg Glu Ala Ser Glu Arg Arg Ser
    1130                    1135                    1140

Ser Asp Ser Tyr Pro Leu Ala Pro Val Arg Ala Pro Arg Thr Leu
    1145                    1150                    1155

Gln Pro Gln His Trp Thr Thr Phe Tyr Lys Pro His Ala Pro Ile
    1160                    1165                    1170

Ile Ser Ile Arg Gly Asn Glu Glu Lys Pro Ala Ser Pro Ser Ala
    1175                    1180                    1185

Ala Val Pro Pro Gly Thr Asp His Asp Pro His Gly Leu Val Val
    1190                    1195                    1200

Lys Ser Met Pro Asp Pro Asp Lys Ala Ser Ala Cys Pro Gly Gln
    1205                    1210                    1215

Ala Thr Gly Gln Pro Lys Glu Asp Ser Glu Glu Leu Gly Leu Pro
    1220                    1225                    1230

Asp Val Asn Pro Met Cys Gln Arg Pro Arg Leu Lys Arg Met Gln
    1235                    1240                    1245

Gln Phe Glu Asp Leu Glu Gly Glu Ile Pro Gln Phe Val
    1250                    1255                    1260

<210>  6
<211>  138
<212>  PRT
<213>  Homo spiens

<400>  6

Met Arg Glu Glu Asn Lys Gly Met Pro Ser Gly Gly Gly Ser Asp Glu
1               5                   10                  15

Gly Leu Ala Ser Ala Ala Ala Arg Gly Leu Val Glu Lys Val Arg Gln

20                  25                  30

Leu Leu Glu Ala Gly Ala Asp Pro Asn Gly Val Asn Arg Phe Gly Arg
        35                  40                  45

Arg Ala Ile Gln Val Met Met Met Gly Ser Ala Arg Val Ala Glu Leu
    50                  55                  60

Leu Leu Leu His Gly Ala Glu Pro Asn Cys Ala Asp Pro Ala Thr Leu
65                  70                  75                  80

Thr Arg Pro Val His Asp Ala Ala Arg Glu Gly Phe Leu Asp Thr Leu
                85                  90                  95

Val Val Leu His Arg Ala Gly Ala Arg Leu Asp Val Arg Asp Ala Trp
            100                 105                 110

Gly Arg Leu Pro Val Asp Leu Ala Glu Glu Arg Gly His Arg Asp Val
        115                 120                 125

Ala Gly Tyr Leu Arg Thr Ala Thr Gly Asp
    130                 135

<210>  7
<211>  168
<212>  PRT
<213>  Homo sapiens

<400>  7

Met Gln Ala Gln Gln Tyr Gln Gln Gln Arg Arg Lys Phe Ala Ala Ala
1               5                   10                  15

Phe Leu Ala Phe Ile Phe Ile Leu Ala Ala Val Asp Thr Ala Glu Ala
            20                  25                  30

Gly Lys Lys Glu Lys Pro Glu Lys Lys Val Lys Lys Ser Asp Cys Gly
        35                  40                  45

Glu Trp Gln Trp Ser Val Cys Val Pro Thr Ser Gly Asp Cys Gly Leu
    50                  55                  60

Gly Thr Arg Glu Gly Thr Arg Thr Gly Ala Glu Cys Lys Gln Thr Met
65                  70                  75                  80

Lys Thr Gln Arg Cys Lys Ile Pro Cys Asn Trp Lys Lys Gln Phe Gly
                85                  90                  95

Ala Glu Cys Lys Tyr Gln Phe Gln Ala Trp Gly Glu Cys Asp Leu Asn
            100                 105                 110

Thr Ala Leu Lys Thr Arg Thr Gly Ser Leu Lys Arg Ala Leu His Asn
        115                 120                 125

Ala Glu Cys Gln Lys Thr Val Thr Ile Ser Lys Pro Cys Gly Lys Leu
    130                 135                 140

Thr Lys Pro Lys Pro Gln Ala Glu Ser Lys Lys Lys Lys Lys Glu Gly
145                 150                 155                 160

Lys Lys Gln Glu Lys Met Leu Asp
                165

<210>  8
<211>  808
<212>  PRT
<213>  Homo sapiens

<400>  8

Met Ala Gly Thr Val Val Leu Asp Asp Val Glu Leu Arg Glu Ala Gln
1               5                   10                  15

Arg Asp Tyr Leu Asp Phe Leu Asp Asp Glu Glu Asp Gln Gly Ile Tyr
            20                  25                  30

Gln Ser Lys Val Arg Glu Leu Ile Ser Asp Asn Gln Tyr Arg Leu Ile
        35                  40                  45

Val Asn Val Asn Asp Leu Arg Arg Lys Asn Glu Lys Arg Ala Asn Arg
    50                  55                  60

Leu Leu Asn Asn Ala Phe Glu Glu Leu Val Ala Phe Gln Arg Ala Leu
65                  70                  75                  80

Lys Asp Phe Val Ala Ser Ile Asp Ala Thr Tyr Ala Lys Gln Tyr Glu
                85                  90                  95

Glu Phe Tyr Val Gly Leu Glu Gly Ser Phe Gly Ser Lys His Val Ser
            100                 105                 110

Pro Arg Thr Leu Thr Ser Cys Phe Leu Ser Cys Val Val Cys Val Glu
        115                 120                 125

Gly Ile Val Thr Lys Cys Ser Leu Val Arg Pro Lys Val Val Arg Ser
    130                 135                 140

Val His Tyr Cys Pro Ala Thr Lys Lys Thr Ile Glu Arg Arg Tyr Ser
145                 150                 155                 160

Asp Leu Thr Thr Leu Val Ala Phe Pro Ser Ser Ser Val Tyr Pro Thr
                165                 170                 175

Lys Asp Glu Glu Asn Asn Pro Leu Glu Thr Glu Tyr Gly Leu Ser Val
            180                 185                 190

Tyr Lys Asp His Gln Thr Ile Thr Ile Gln Glu Met Pro Glu Lys Ala

195                 200                 205

Pro Ala Gly Gln Leu Pro Arg Ser Val Asp Val Ile Leu Asp Asp Asp
    210                 215                 220

Leu Val Asp Lys Ala Lys Pro Gly Asp Arg Val Gln Val Val Gly Thr
225                 230                 235                 240

Tyr Arg Cys Leu Pro Gly Lys Lys Gly Gly Tyr Thr Ser Gly Thr Phe
                245                 250                 255

Arg Thr Val Leu Ile Ala Cys Asn Val Lys Gln Met Ser Lys Asp Ala
            260                 265                 270

Gln Pro Ser Phe Ser Ala Glu Asp Ile Ala Lys Ile Lys Lys Phe Ser
        275                 280                 285

Lys Thr Arg Ser Lys Asp Ile Phe Asp Gln Leu Ala Lys Ser Leu Ala
    290                 295                 300

Pro Ser Ile His Gly His Asp Tyr Val Lys Lys Ala Ile Leu Cys Leu
305                 310                 315                 320

Leu Leu Gly Gly Val Glu Arg Asp Leu Glu Asn Gly Ser His Ile Arg
                325                 330                 335

Gly Asp Ile Asn Ile Leu Leu Ile Gly Asp Pro Ser Val Ala Lys Ser
            340                 345                 350

Gln Leu Leu Arg Tyr Val Leu Cys Thr Ala Pro Arg Ala Ile Pro Thr
        355                 360                 365

Thr Gly Arg Gly Ser Ser Gly Val Gly Leu Thr Ala Ala Val Thr Thr
    370                 375                 380

Asp Gln Glu Thr Gly Glu Arg Arg Leu Glu Ala Gly Ala Met Val Leu
385                 390                 395                 400

Ala Asp Arg Gly Val Val Cys Ile Asp Glu Phe Asp Lys Met Ser Asp
                405                 410                 415

Met Asp Arg Thr Ala Ile His Glu Val Met Glu Gln Gly Arg Val Thr
            420                 425                 430

Ile Ala Lys Ala Gly Ile His Ala Arg Leu Asn Ala Arg Cys Ser Val
        435                 440                 445

Leu Ala Ala Ala Asn Pro Val Tyr Gly Arg Tyr Asp Gln Tyr Lys Thr
    450                 455                 460

Pro Met Glu Asn Ile Gly Leu Gln Asp Ser Leu Leu Ser Arg Phe Asp
465                 470                 475                 480

Leu Leu Phe Ile Met Leu Asp Gln Met Asp Pro Glu Gln Asp Arg Glu
                485                 490                 495

Ile Ser Asp His Val Leu Arg Met His Arg Tyr Arg Ala Pro Gly Glu
            500                 505                 510

Gln Asp Gly Asp Ala Met Pro Leu Gly Ser Ala Val Asp Ile Leu Ala
        515                 520                 525

Thr Asp Asp Pro Asn Phe Ser Gln Glu Asp Gln Gln Asp Thr Gln Ile
    530                 535                 540

Tyr Glu Lys His Asp Asn Leu Leu His Gly Thr Lys Lys Lys Lys Glu
545                 550                 555                 560

Lys Met Val Ser Ala Ala Phe Met Lys Lys Tyr Ile His Val Ala Lys
                565                 570                 575

Ile Ile Lys Pro Val Leu Thr Gln Glu Ser Ala Thr Tyr Ile Ala Glu
            580                 585                 590

Glu Tyr Ser Arg Leu Arg Ser Gln Asp Ser Met Ser Ser Asp Thr Ala
        595                 600                 605

Arg Thr Ser Pro Val Thr Ala Arg Thr Leu Glu Thr Leu Ile Arg Leu
    610                 615                 620

Ala Thr Ala His Ala Lys Ala Arg Met Ser Lys Thr Val Asp Leu Gln
625                 630                 635                 640

Asp Ala Glu Glu Ala Val Glu Leu Val Gln Tyr Ala Tyr Phe Lys Lys
                645                 650                 655

Val Leu Glu Lys Glu Lys Lys Arg Lys Lys Arg Ser Glu Asp Glu Ser
            660                 665                 670

Glu Thr Glu Asp Glu Glu Glu Lys Ser Gln Glu Asp Gln Glu Gln Lys
        675                 680                 685

Arg Lys Arg Arg Lys Thr Arg Gln Pro Asp Ala Lys Asp Gly Asp Ser
    690                 695                 700

Tyr Asp Pro Tyr Asp Phe Ser Asp Thr Glu Glu Glu Met Pro Gln Val
705                 710                 715                 720

His Thr Pro Lys Thr Ala Asp Ser Gln Glu Thr Lys Glu Ser Gln Lys
                725                 730                 735

Val Glu Leu Ser Glu Ser Arg Leu Lys Ala Phe Lys Val Ala Leu Leu
            740                 745                 750

Asp Val Phe Arg Glu Ala His Ala Gln Ser Ile Gly Met Asn Arg Leu
        755                 760                 765

Thr Glu Ser Ile Asn Arg Asp Ser Glu Glu Pro Phe Ser Ser Val Glu
    770                 775                 780

Ile Gln Ala Ala Leu Ser Lys Met Gln Asp Asp Asn Gln Val Met Val
785                 790                 795                 800

Ser Glu Gly Ile Ile Phe Leu Ile
                805

<210>  9
<211>  20
<212>  DNA
<213>  artificial

<220>
<223>  primer

<400>  9
ttcgtcatgg gtgtgaacca                                                   20

<210>  10
<211>  21
<212>  DNA
<213>  artificial

<220>
<223>  primer

<400>  10
ctgtggtcat gagtccttcc a                                                 21

<210>  11
<211>  20
<212>  DNA
<213>  artificial

<220>
<223>  primer

<400>  11
caatgatcat gcccagtgca                                                   20

<210>  12
<211>  20
<212>  DNA
<213>  artificial

<220>
<223>  primer

<400>  12
gatcgtgatc tgtgccagga                                                   20

<210>  13
<211>  20
<212>  DNA
<213>  artificial

<220>
<223>  primer

<400>  13
cccaactcca ccagatagca                                                   20


<210>  14
<211>  20
<212>  DNA
<213>  artificial

<220>
<223>  primer

<400>  14
gggatttccg catcctagca                                                   20


<210>  15
<211>  20
<212>  DNA
<213>  artificial

<220>
<223>  primer

<400>  15
cagtgagtca tccgtccaga                                                   20


<210>  16
<211>  20
<212>  DNA
<213>  artificial

<220>
<223>  primer

<400>  16
gccattctcc acagtcagac                                                   20


<210>  17
<211>  20
<212>  DNA
<213>  artificial

<220>
<223>  primer

<400>  17
acattgggct acaggactca                                                   20


<210>  18
<211>  20
<212>  DNA
<213>  artificial

<220>
<223>  primer

<400>  18
tgaatgctgc actcaccatc                                                   20